# EUROPEAN PATENT APPLICATION

(11) **EP 4 095 159 A1**
(43) Date of publication of application: **30.11.2022**
(21) Application number: 21744582.4
(22) Date of filing: 19.01.2021
(51) Int. Cl.: C07K 16/28, C12N 15/13, C12N 15/63, C12N 5/10, A61K 39/395, A61P 35/00

(54) **ANTI-LAG3 MONOCLONAL ANTIBODY, AND PREPARATION METHOD THEREFOR AND USE THEREOF**

(30) Priority: 21.01.2020 CN 202010069008
(71) Applicant: Shanghai Henlius Biotech, Inc., Shanghai 201210 (CN); Shanghai Henlius Biopharmaceutical Co., Ltd., Shanghai 200233 (CN)
(72) Inventor: SONG, Ge, Shanghai 201210 (CN); XIAO, Hui, Shanghai 201210 (CN); HE, Honglin, Shanghai 201210 (CN); XU, Xu, Shanghai 201210 (CN); JIANG, Wei-Dong, Shanghai 201210 (CN)
(74) Representative: Rentsch Partner AG
(86) International application number: PCT/CN2021/072610
(87) International publication number: WO 2021/147837

(57) **Abstract**

Provided in the present invention are an anti-LAG3 monoclonal antibody and the use thereof. Further provided are a nucleotide molecule for encoding the antibody, an expression vector and a host cell for expressing the antibody, a composition containing the antibody, and the use of the antibody in the preparation of drugs for resisting tumors, treating autoimmune diseases, and treating infectious diseases and/or resisting transplantation rejection reactions.

## Description

### Technical Field

The present invention relates to the field of biomedicine, in particular to an anti-LAG3 monoclonal antibody, a preparation method therefor and the use thereof.

### Background Art

LAG3 (Lymphocyte-activation gene 3), also known as CD223, was discovered in 1990. LAG3 belongs to an immunoglobulin superfamily and is expressed on activated T cells, NK cells, B cells and dendritic cells. One of the ligands of LAG3 is the major histocompatibility complex (MHC) class II molecules. The affinity of LAG3 for MHC II is even stronger than that of CD4 for MHC II. Moreover, the ligands of LAG3 also include LSECtin, FGL1, etc. LAG3, whose biological function is similar to that of CTLA-4 and PD-1, plays a negative regulatory role in T cell proliferation and activation. Moreover, LAG3 also plays a role in the suppressive function of Treg cells.

Studies have found that T cells can be reactivated by inhibiting LAG3, thereby enhancing the killing effect on tumors. Meanwhile, the function of Treg cells to suppress the immune response can also be reduced by inhibiting LAG3. Therefore, using LAG3 as a target for cancer immunotherapy and using a monoclonal antibody to bind to LAG3 can block the binding of LAG3 to the ligands thereof and prevent the production of T cell inhibitory signals, thereby promoting T cell activation and proliferation and cytokine expression, up-regulating the monitoring activity of the immune system against tumor cells, enhancing the specific anti-tumor immune response, and achieving the purpose of treating tumors. At present, pharmaceutical companies including Bristol-Myers Squibb, Novartis, and Merck are all developing their own antibody drugs against LAG3, which are currently in the clinical trial phase. The anti-LAG3 antibody BMS986016 (Relatlimab), researched and developed by Bristol-Myers Squibb Company, has achieved significant effects in the treatment of advanced melanoma, and is currently undergoing a phase III clinical trial. Moreover, anti-LAG3 antibodies are used in combination with other antibody drugs comprising anti-PD-1 antibodies, and relevant clinical trials are also being carried out.

Therefore, the research and development of a novel anti-LAG3 monoclonal antibody with lower toxic and side effects and better clinical efficacy for cancer immunotherapy has become a current research hotspot, and will also provide patients with more drug options.

### Summary of the Invention

The technical problem to be solved by the present invention is to provide a novel anti-LAG3 monoclonal antibody, thereby completing the present invention.

Therefore, the first objective of the present invention is to provide a novel anti-LAG3 monoclonal antibody.

The second objective of the present invention is to provide a nucleotide molecule encoding the anti-LAG3 monoclonal antibody.

The third objective of the present invention is to provide an expression vector comprising the nucleotide molecule.

The fourth objective of the present invention is to provide a host cell comprising the expression vector.

The fifth objective of the present invention is to provide a method for preparing the anti-LAG3 monoclonal antibody.

The sixth objective of the present invention is to provide a composition comprising the anti-LAG3 monoclonal antibody.

The seventh objective of the present invention is to provide the use of the anti-LAG3 monoclonal antibody in the preparation of drugs.

In order to achieve the above-mentioned objectives, the present invention uses the following technical solutions:

the first aspect of the present invention provides an anti-LAG3 monoclonal antibody, wherein the monoclonal antibody comprises a heavy chain variable region comprising HCDR1, HCDR2 and HCDR3 identical to CDR sequences of the heavy chain variable region as shown in SEQ ID NO: 2, 6 or 10, and a light chain variable region comprising LCDR1, LCDR2 and LCDR3 identical to CDR sequences of the light chain variable region as shown in SEQ ID NO: 4, 8 or 12.

In some embodiments, the anti-LAG3 monoclonal antibody comprises:
1) heavy chain complementarity determining regions: HCDR1 having an amino acid sequence as shown in SEQ ID No: 18, HCDR2 having an amino acid sequence as shown in SEQ ID No: 19, and HCDR3 having an amino acid sequence as shown in SEQ ID No: 20 or 21; and
2) light chain complementarity determining regions: LCDR1 having an amino acid sequence as shown in SEQ ID No: 22 or 23, LCDR2 having an amino acid sequence as shown in SEQ ID No: 24, and LCDR3 having an amino acid sequence as shown in SEQ ID No: 25.

In some embodiments, the anti-LAG3 monoclonal antibody comprises a heavy chain variable region and a light chain variable region, wherein the heavy chain variable region has an amino acid sequence as shown in SEQ ID NO: 2, 6 or 10, or a sequence with at least 85% homology, such as a derived sequence with 85%, 90%, 92%, 94%, 95%, 96%, 97%, 98% or 99% homology thereto; and the light chain variable region has an amino acid sequence as shown in SEQ ID NO: 4, 8 or 12, or a sequence with at least 85% homology, such as a derived sequence with 85%, 90%, 92%, 94%, 95%, 96%, 97%, 98% or 99% homology thereto.

In some embodiments, the anti-LAG3 monoclonal antibody comprises a heavy chain and a light chain, wherein the heavy chain is composed of a heavy chain variable region having an amino acid sequence as shown in SEQ ID NO: 2, 6 or 10, or a sequence with at least 85% homology, such as a sequence with 85%, 90%, 92%, 94%, 95%, 96%, 97%, 98% or 99% homology thereto, and a heavy chain constant region as shown in SEQ ID NO: 14; and the light chain is composed of a light chain variable region having an amino acid sequence as shown in SEQ ID NO: 4, 8 or 12, or a sequence with at least 85% homology, such as a sequence with 85%, 90%, 92%, 94%, 95%, 96%, 97%, 98% or 99% homology thereto, and a light chain constant region as shown in SEQ ID NO: 16.

Preferably, the anti-LAG3 monoclonal antibody comprises a heavy chain variable region having an amino acid sequence as shown in SEQ ID NO: 2, and a light chain variable region having an amino acid sequence as shown in SEQ ID NO: 4.

Preferably, the anti-LAG3 monoclonal antibody comprises a heavy chain variable region having an amino acid sequence as shown in SEQ ID NO: 6, and a light chain variable region having an amino acid sequence as shown in SEQ ID NO: 8.

Preferably, the anti-LAG3 monoclonal antibody comprises a heavy chain variable region having an amino acid sequence as shown in SEQ ID NO: 10, and a light chain variable region having an amino acid sequence as shown in SEQ ID NO: 12.

The anti-LAG3 monoclonal antibody may be a full-length sequence of the anti-LAG3 antibody or a fragment thereof, wherein the fragment is Fab, Fab', F(ab')2, Fv or scFv. Preferably, the anti-LAG3 antibody is an IgG1, IgG2 or IgG4 antibody.

The present invention further provides a derivative of the anti-LAG3 antibody, wherein the derivative is a fragment of the LAG3 antibody, an antibody/antibody fragment-factor fusion protein, or an antibody/antibody fragment-chemical conjugate; and the fragment of the anti-LAG3 antibody is Fab, Fab', F(ab')2, Fv or scFv.

The monoclonal antibody of the present invention can be prepared by conventional techniques in the art, including a hybridoma technique, phage display technique, single lymphocyte gene cloning technique, etc. Preferably, the monoclonal antibody is prepared from wild-type or transgenic mice by the hybridoma technique.

The second aspect of the present invention provides an isolated nucleotide molecule encoding the anti-LAG3 monoclonal antibody as described above.

In some embodiments, the nucleotide sequence encoding the heavy chain variable region of the anti-LAG3 monoclonal antibody is as shown in SEQ ID NO: 3, 7 or 11, and the nucleotide sequence encoding the light chain variable region of the anti-LAG3 monoclonal antibody is as shown in SEQ ID NO: 5, 9 or 13.

Preferably, the nucleotide sequence encoding the heavy chain variable region of the anti-LAG3 monoclonal antibody is as shown in SEQ ID NO: 3, and the nucleotide sequence encoding the light chain variable region of the anti-LAG3 monoclonal antibody is as shown in SEQ ID NO: 5.

Preferably, the nucleotide sequence encoding the heavy chain variable region of the anti-LAG3 monoclonal antibody is as shown in SEQ ID NO: 7, and the nucleotide sequence encoding the light chain variable region of the anti-LAG3 monoclonal antibody is as shown in SEQ ID NO: 9.

Preferably, the nucleotide sequence encoding the heavy chain variable region of the anti-LAG3 monoclonal antibody is as shown in SEQ ID NO: 11, and the nucleotide sequence encoding the light chain variable region of the anti-LAG3 monoclonal antibody is as shown in SEQ ID NO: 13.

The method for preparing the nucleotide molecule is a conventional preparation method in the art, preferably including the following preparation method: using a gene cloning technique, such as a PCR method, to obtain the nucleotide molecule encoding the monoclonal antibody, or using an artificial whole sequence synthetic method to obtain the nucleotide molecule encoding the above-mentioned monoclonal antibody.

It is known to those skilled in the art that a substitution, deletion, alteration, insertion or addition can be appropriately introduced into a nucleotide sequence encoding an amino acid sequence of the above-mentioned monoclonal antibody to provide a homolog of a polynucleotide or a conserved variation sequence thereof. The homolog of the polynucleotide or the conserved variation sequence thereof in the present invention can be prepared by the substitution, deletion, or addition of one or more bases of the gene encoding the monoclonal antibody within the range of maintaining the antibody activity.

The third aspect of the present invention provides an expression vector containing the nucleotide molecule as described above.

The expression vector is a conventional expression vector in the art, and refers to an expression vector comprising appropriate regulatory sequences, such as promoter sequences, terminator sequences, polyadenylation sequences, enhancer sequences, marker genes and/or sequences, and other appropriate sequences. The expression vector may be a virus or plasmid, such as an appropriate phage or phagemid; for more technical details, reference can be made to, for example, Sambrook et al., Molecular Cloning: A Laboratory Manual, 2nd Edition, Cold Spring Harbor Laboratory Press, 1989. For many known techniques and protocols for nucleic acid manipulation, reference can be made to Current Protocols in Molecular Biology, 2nd Edition, edited by Ausubel et al. The expression vector in the present invention refers to a bacterial plasmid, phage, yeast plasmid, plant cell virus, mammalian cell virus such as adenovirus, retrovirus, or other vectors well known in the art.

Preferably, the expression vector is selected from one or more of pHLX101, pEE14.4, pCHO 1.0 or pcDNA3.1.

More preferably, the expression vector is pcDNA3.1.

The fourth aspect of the present invention provides a host cell containing the expression vector as described above.

The host cell of the present invention is various conventional host cells in the art, as long as it meets the following requirements: in the host cell, the above-mentioned recombinant expression vector can be stably replicated by oneself, and the nucleotides carried thereby can be effectively expressed. The host cell in the present invention can be prokaryotic cells, such as bacterial cells; or lower eukaryotic cells, such as yeast cells; or higher eukaryotic cells, such as mammalian cells.

Preferably, the host cell is one or more of COS, CHO (Chinese Hamster Ovary), an HeLa cell line, a myeloid cell lines such as an SP2/0 cell line, NS0, sf9, sf21, DH5α, BL21(DE3) or E.coli TG1, a YB2/0 cell line and a transformed B cell or a hybridoma cell.

More preferably, the host cell is E.coli TG1 or BL21 cells (expressing a single chain antibody or Fab antibody) or CHO-K1 cells (expressing a full-length IgG antibody).

The preferred recombinant expression transformant of the present invention can be obtained by transforming the expression vector into a host cell. The transformation method is a conventional transformation method in the art, preferably a chemical transformation method, a heat shock method or an electroporation method.

The fifth aspect of the present invention provides a method for preparing the anti-LAG3 monoclonal antibody as described above, comprising the following steps:
a) culturing the host cell as described above to express the anti-LAG3 monoclonal antibody under an expression condition; and
b) isolating and purifying the anti-LAG3 monoclonal antibody obtained in step a).

The method for culturing the host cell and the method for isolating and purifying the anti-LAG3 monoclonal antibody of the present invention are conventional methods in the art; for the specific operation method, reference can be made to the corresponding cell culture technical handbook and monoclonal antibody isolation and purification technical handbook.

The host cells used in the present invention are all those in the prior art, and are directly commercially available. The culture media used in a culture process are various conventional culture media. Those skilled in the art may select applicable culture media according to experience for culture under the condition suitable for the growth of the host cells. The selected promoter may be induced using an appropriate method (e.g., temperature changes or chemical induction) when host cells are grown to an appropriate density, and the cells are cultured for an additional period. Recombinant polypeptides in the above-mentioned methods can be expressed inside the cells or on the cell membranes, or secreted outside the cells. If necessary, the recombinant proteins can be isolated and purified by various isolation methods using the physical, chemical and other properties thereof. These methods are well known to those skilled in the art. Examples of these methods include but are not limited to: conventional renaturation treatment, treatment by a protein precipitant (salt precipitation), centrifugation, cell lysis by osmosis, sonication, supercentrifugation, molecular sieve chromatography (gel filtration), adsorption chromatography, ion exchange chromatography, high performance liquid chromatography (HPLC), and any other liquid chromatography, and a combination thereof.

In the present invention, the cell line cultured from a monoclone is screened to obtain the gene sequence of an antibody of interest, which is used to construct a eukaryotic expression vector, and after expression, the activity of the antibody can be reconstructed to obtain an anti-LAG3 monoclonal antibody.

The sixth aspect of the present invention provides a composition comprising the anti-LAG3 monoclonal antibody as described above and a pharmaceutically acceptable carrier.

The monoclonal antibody of the present invention can be used in a pharmaceutical composition formulated in any manner known in the art. Such composition contains the monoclonal antibody as an active ingredient, together with one or more pharmaceutically acceptable carriers, diluents, fillers, binders and other excipients, depending on the mode of administration and the dosage form contemplated. Therapeutically inert inorganic or organic carriers known to those skilled in the art include, but are not limited to, lactose, corn starch or derivatives thereof, talc, vegetable oils, waxes, fats, polyhydroxy compounds such as polyethylene glycol, water, sucrose, ethanol, glycerol and the like, various preservatives, lubricants, dispersants, and flavoring agents. Humectants, antioxidants, sweeteners, colorants, stabilizers, salts, buffers, and the like may also be added to the composition. These substances, as required, are used to assist in the stability of the formulation or to help to increase the activity or bioavailability thereof, or to confer an acceptable taste or odor thereto in the case of oral dosing. In such composition, an inhibitor can be used in the form of the original compound itself, or optionally in the form of a pharmaceutically acceptable salt thereof. The monoclonal antibody of the present invention can be administered alone, or in various combinations, and in conjunction with other therapeutic agents. The inhibitor can be administered in the composition so formulated by any suitable manner known to those skilled in the art, as required.

The anti-LAG3 monoclonal antibody provided by the present invention can be combined with a pharmaceutically acceptable carrier to form a pharmaceutical preparation composition, so as to exert a more stable efficacy. These preparations can ensure the conformational integrity of the amino acid core sequence of the anti-LAG3 monoclonal antibody of the present invention, and also protect the multifunctional groups of the protein from degradation (including but not limited to aggregation, deamination or oxidation). Typically, liquid preparations are stable at 2°C-8°C for at least one year, and lyophilized preparations are stable at 30°C for at least six months. The anti-LAG3 monoclonal antibody preparation may be a suspension preparation, a water injection preparation, a lyophilized preparation, etc. commonly used in the pharmaceutical field, preferably a water injection preparation or a lyophilized preparation.

For the water injection preparation or the lyophilized preparation of the anti-LAG3 monoclonal antibody of the present invention, a pharmaceutically acceptable carrier preferably includes, but is not limited to: one of surfactants, solution stabilizers, isoosmotic regulators and buffers, or a combination thereof. The surfactants preferably include, but are not limited to: non-ionic surfactants such as polyoxyethylene sorbitan fatty acid ester (Tween 20 or 80); Poloxamer (e.g., Poloxamer 188); Triton; sodium dodecyl sulfate (SDS); sodium lauryl sulfate; tetradecyl, linoleyl or octadecyl sarcosine; Pluronics; MONAQUATTM, etc. The surfactants should be added in an amount such that the tendency for the anti-LAG3 monoclonal antibody to granulation is minimized. The solution stabilizers preferably include, but are not limited to, one of the following: sugars, such as reducing and non-reducing sugars; amino acids, such as monosodium glutamate or histidine; and alcohols, such as trihydric alcohols, higher sugar alcohols, propylene glycol and polyethylene glycol, or a combination thereof. The solution stabilizers should be added in an amount such that the final formed preparation remains stable for a period of time that is considered stable by those skilled in the art. The isoosmotic regulators preferably include, but are not limited to, one of sodium chloride and mannitol, or a combination thereof. The buffers preferably include, but are not limited to: one of Tris, histidine buffer and phosphate buffer, or a combination thereof.

The seventh aspect of the present invention provides the use of the anti-LAG3 monoclonal antibody or the composition thereof as described above in the preparation of drugs.

Preferably, the use is in the preparation of an LAG3 molecule blocking drug. More preferably, the use in the preparation of an LAG3 molecule blocking drug specifically comprises preparing a tumor therapeutic or tumor diagnostic drug.

The drugs of the present invention are preferably drugs for resisting tumors, treating autoimmune diseases, and treating infectious diseases and/or resisting transplantation rejection reactions, more preferably drugs for resisting tumors or drugs for treating autoimmune diseases, and further preferably drugs for resisting tumors. The anti-LAG3 monoclonal antibody of the present invention can be used alone or in combination with other drugs for resisting tumors. The other drugs for resisting tumors are conventional drugs for resisting tumors in the art, including an antibody drug or a small-molecule drug for resisting tumors. The antibody drugs are conventional antibody drugs in the art, preferably including an anti-PD-1 monoclonal antibody. The small-molecule drugs for resisting tumors are conventional drugs in the art, including paclitaxel, 5-fluorouracil, etc.

The tumors targeted by the drugs for resisting tumors preferably include, but are not limited to: one or more of melanoma, lung cancer, liver cancer, ovarian cancer, cervical cancer, skin cancer, colon cancer, glioma, bladder cancer, breast cancer, kidney cancer, esophageal cancer, gastric cancer, oral squamous cell carcinoma, urothelial cell carcinoma, pancreatic cancer and/or head and neck tumor.

The drugs for resisting tumors of the present invention refer to drugs that inhibit and/or treat tumors, which may include the delay in the development of symptoms associated with tumors and/or the reduction in the severity of these symptoms, and also include the alleviation of symptoms associated with existing tumors and the prevention of the other symptoms, and further include the reduction or prevention of metastasis of the tumors.

When the anti-LAG3 monoclonal antibody and the composition thereof in the present invention are administered to animals including human, the administration dose varies depending on the age and weight of a patient, the characteristics and severity of a disease, and the administration route. For the administration dose, reference can be made to the results of the animal experiment and various conditions, and the total dose cannot exceed a certain range. Generally, the dose for intravenous injection is 1-1800 mg/day.

On the basis of conforming to common knowledge in the art, the above-mentioned preferred conditions can be arbitrarily combined to obtain various preferred embodiments of the present invention.

Beneficial effects of the present invention are as follows:

the anti-LAG3 monoclonal antibody of the present invention has a good biological activity, has a relatively high expression level in mammalian cells, and has an obvious affinity for LAG3 and the ability to inhibit ligand-receptor binding. The monoclonal antibody can be used alone or in combination with other drugs for resisting tumors in tumor immunotherapy, diagnosis and screening, and can be effectively used in the preparation of drugs for treating tumors, infectious diseases, autoimmune diseases and anti-immune rejection drugs.

### Brief Description of the Drawings

Figure 1 shows experimental results of binding ability of the anti-LAG3 antibody to LAG3 on the cell surface.
Figure 2 shows experimental results of the anti-LAG3 antibody blocking the binding of LAG3 to the ligand MHC II thereof.
Figure 3 shows experimental results of binding ability of the anti-LAG3 chimeric antibody to LAG3.
Figure 4 shows experimental results of binding ability of the anti-LAG3 chimeric antibody to LAG3 on the cell surface.
Figure 5 shows experimental results of binding ability of the anti-LAG3 humanized antibody to LAG3.
Figure 6 shows experimental results of binding ability of the anti-LAG3 humanized antibody to LAG3 on the cell surface.
Figure 7 shows surface plasmon resonance experimental results of affinity of the anti-LAG3 humanized antibody for LAG3.
Figure 8 shows experimental results of the anti-LAG3 humanized antibody blocking the binding of LAG3 to the ligand MHC II thereof.
Figure 9 shows experimental results of the anti-LAG3 humanized antibody blocking the binding of LAG3 to the ligand LSECtin thereof.
Figure 10 shows experimental results of the anti-LAG3 humanized antibody blocking the binding of LAG3 to the ligand FGL1 thereof.
Figure 11 shows ELISA experimental results of species cross-reactivity of the anti-LAG3 humanized antibody.
Figure 12 shows flow cytometry experimental results of species cross-reactivity of the anti-LAG3 humanized antibody.
Figure 13 shows surface plasmon resonance experimental results of species cross-reactivity of the anti-LAG3 humanized antibody.
Figure 14 shows experimental results of T cell activation by the anti-LAG3 humanized antibody.
Figure 15 shows experimental results of tumor growth inhibition in the hLAG3 KI mouse model by the anti-LAG3 humanized antibody.
Figure 16 shows that h6H11B10#40 is administered in combination with HLX10 to activate the release of IL-2.
Figure 17 shows that h6H11B10#40 is administered in combination with HLX10 to activate the release of IFN-γ.
Figure 18 shows the growth curve of the tumor volume in each group of mice in the MC38 model.
   Notes: data is expressed as "mean ± standard error".
Figure 19 shows the growth curve of the tumor volume in each group of mice in the A20 model.
   Notes: 1. data is expressed as "mean ± standard error".

### Detailed Description of Embodiments

The present invention is further illustrated by way of examples below; however, the present invention is not limited to the scope of the described examples. For the experimental methods in which no specific conditions are specified in the examples, selections are made according to conventional methods and conditions or according to the product instructions. The room temperature described in the examples is a conventional room temperature in the art, generally 10°C-30°C. Unless otherwise specified, the reagents and raw materials used are commercially available.

It should be understood that the scope of protection of the present invention is not limited to the following particular specific embodiments; and the terms used in the examples of the present invention are intended to describe the specific embodiments of the present invention, rather than limiting the scope of protection of the present invention. In the specification and claims of the present invention, unless otherwise clearly stated in the context, the singular forms "a", "an", and "the" include the plural forms.

The term "variable region" of an antibody refers to a light chain variable region (VL) of an antibody or a heavy chain variable region (VH) of an antibody, either alone or in combination. As known in the art, each of the heavy and light chain variable regions consists of four framework regions (FRs) connected by three complementarity determining regions (CDRs) (also known as hypervariable regions). The CDRs in each chain are held together in close proximity by the FRs, and together with the CDRs from another chain contribute to the formation of the antigen-binding site of the antibody. There are at least two techniques for determining CDRs: (1) a method based on cross-species sequence variability (i.e., Kabat et al., Sequences of Proteins of Immunological Interest, 5th Edition, 1991, National Institutes of Health, Bethesda MD); and (2) a method based on crystallographic studies of antigen-antibody complexes (Al-Lazikani et al., J. Molec. Biol. 273:927-948 (1997)). As used herein, CDRs may refer to those determined by either method or by a combination of the two methods.

The term "antibody framework" or "FR region" refers to a moiety of the variable domain VL or VH, which serves as a scaffold for the antigen binding loops (CDRs) of the variable domain. Essentially, it is a variable domain without CDRs.

The terms "complementarity determining region" and "CDR" refer to one of six hypervariable regions in the variable domain of an antibody that mainly contribute to antigen binding. Typically, there are three CDRs in each heavy chain variable region (CDR-H1, CDR-H2, and CDR-H3) and three CDRs in each light chain variable region (CDR-L1, CDR-L2, and CDR-L3). The amino acid sequence boundaries of CDRs can be determined using any of a variety of well-known schemes, including "Kabat" numbering rule (see Kabat et al. (1991), "Sequences of Proteins of Immunological Interest", 5th Edition, Public Health Service, National Institutes of Health, Bethesda, MD), "Chothia" numbering rule (Al-Lazikani et al. (1997), JMB 273:927-948), ImMunoGenTics (IMGT) numbering rule (Lefranc M.P., Immunologist, 7, 132-136 (1999); Lefranc, M.P. et al., Dev. Comp. Immunol., 27, 55-77 (2003)), etc. For example, for the classical format, following the Kabat rule, CDR amino acid residues in the heavy chain variable domain (VH) are numbered 31-35 (HCDR1), 50-65 (HCDR2) and 95-102 (HCDR3); and CDR amino acid residues in the light chain variable domain (VL) are numbered 24-34 (LCDR1), 50-56 (LCDR2) and 89-97 (LCDR3). Following the Chothia rule, CDR amino acids in VH are numbered 26-32 (HCDR1), 52-56 (HCDR2) and 95-102 (HCDR3); and amino acid residues in VL are numbered 26-32 (LCDR1), 50-52 (LCDR2) and 91-96 (LCDR3). By combining the CDR definitions of both Kabat and Chothia, CDRs consist of amino acid residues 26-35 (HCDR1), 50-65 (HCDR2) and 95-102 (HCDR3) in human VH and amino acid residues 24-34 (LCDR1), 50-56 (LCDR2) and 89-97 (LCDR3) in human VL. Following the IMGT rule, CDR amino acid residues in VH are numbered approximately 26-35 (HCDR1), 51-57 (HCDR2) and 93-102 (HCDR3); and CDR amino acid residues in VL are numbered approximately 27-32 (LCDR1), 50-52 (LCDR2) and 89-97 (LCDR3). Following the IMGT rule, the CDRs of an antibody can be determined using the program IMGT/DomainGap Align.

The term "LAG3" refers to the lymphocyte activation gene-3 protein, an immune checkpoint receptor or T cell co-inhibitor, also known as CD223. The amino acid sequence of the full-length LAG3 is provided in GenBank under the accession number NP_002277.4. The term "" includes variants, isoforms, homologs, orthologs and paralogs, such as LAG3 protein variants, recombinant LAG3 or fragments thereof, and LAG3 or fragments thereof coupled to, for example, a histidine tag, mouse or human Fc or signal sequence (e.g., ROR1).

When numerical ranges are given in the examples, unless otherwise indicated in the present invention, the two endpoints of each numerical range and any value between the two endpoints can be selected. Unless defined otherwise, all technical and scientific terms used in the present invention have the same meanings commonly understood by those skilled in the art. In addition to the specific methods, devices, and materials used in the examples, according to the knowledge in the prior art and the description of the present invention, those skilled in the art can also use any prior art methods, devices, and materials which are similar or equivalent to the methods, devices, and materials described in the examples of the present invention to realize the present invention.

Unless otherwise specified, the experimental methods, detection methods and preparation methods disclosed in the present invention all use conventional molecular biology, biochemistry, chromatin structure and analysis, analytical chemistry, cell culture, recombinant DNA techniques in the art and other conventional techniques in related arts. The techniques have been completely described in existing documents. For details, reference can be made to: Sambrook et al., MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, Chromatin (P.M.Wassarman and A.P.Wolffe, eds.), Academic Press, San Diego, 1999; and METHODS IN MOLECULAR BIOLOGY, Vol. 119, Chromatin Protocols (P.B.Becker, ed.) Humana Press, Totowa, 1999.

Positive control antibody BMS986016: the amino acid sequence thereof is disclosed in the patent application WO 2015/042246 A1 of Bristol-Myers Squibb Company. In the present invention, the nucleotide sequence encoding the amino acid sequence is cloned into a pURO vector using a restriction endonuclease, and then the plasmid is transfected into CHO-S cells which are screened according to carbenicillin resistance to obtain a high-expression cell line. The cell line is expanded and cultured, the supernatant of the culture medium is collected, and the control antibody is obtained by a protein A affinity chromatography preparation process.
Negative control HLX10 (h1G4): the sequence thereof is cited from CN109923126A.
Positive control ab40465: Abcam, Cat. No. ab40465.
Isotype - Human IgG4 (hIgG4): CrownBio, Cat: AB180018.
Recombinant mouse LAG3-Fc fusion protein: purchased from Sino Biological, Cat: 53069-M02H.
Recombinant cynomolgus monkey LAG3-Fc fusion protein: purchased from Sino Biological, Cat: 90841-C08H.
Anti-mouse PD1 antibody RMP1-14: BioXCell, Cat: 71791801

### Example 1 Screening of the anti-LAG3 antibody

In this example, mice are co-immunized with an LAG3-His protein (the amino acid sequence thereof as shown in SEQ ID No: 1) that is house-constructed, expressed and purified, and CHO-S cells expressing a full-length LAG3; spleen cells are fused with myeloma cells to obtain hybridoma cells, and then positive clones are screened using ELISA.

Specifically, a nucleic acid sequence encoding amino acids 23-450 of the LAG3 extracellular region is firstly cloned into a pEE14.4 vector using a restriction endonuclease, wherein the nucleic acid sequence contains a sequence encoding 6xHis-tag in the C-terminus of the LAG3 coding region, and then the plasmid is electroporated into CHO-S cells; monoclones with high expression of the LAG3-his fusion protein are screened out by a GS (Glutamine Synthetase) screening system, and expanded and cultured; the supernatant of the culture medium is collected, and the target protein is prepared by a nickel-column affinity chromatography method. Subsequently, mice are co-immunized with the protein and the CHO-S cells expressing the full-length LAG3. In the process of screening positive hybridomas, a 96-well half-well plate is firstly coated with an LAG3-His recombinant protein at 2 µg/ml (a working volume of 30 µl), and then coated overnight at 4°C. Excess LAG3-His is washed away by washing 3 times with PBS containing 0.05% Tween 20 (PBST). The plate is blocked with PBS containing 5% skimmed milk for 1 hour at room temperature. The plate is washed 3 times with PBST, and then the diluted hybridoma cell culture supernatant is added thereto; the reaction is allowed to stand for 1 hour at room temperature, and then the plate is washed 3 times with PBST; 30 µl of the horseradish peroxidase-labeled goat anti-mouse IgG secondary antibody (CoWin Biosciences) diluted with PBS is added to the plate, with a dilution ratio of 1 : 2000; and after standing at room temperature for 1 hour, the plate is washed 6 times with PBST, and TMB is added thereto for color development; and the reaction is stopped with 30 µl of 2M H₂SO₄. The light absorption at a wavelength of 450 nm is read with a microplate reader.

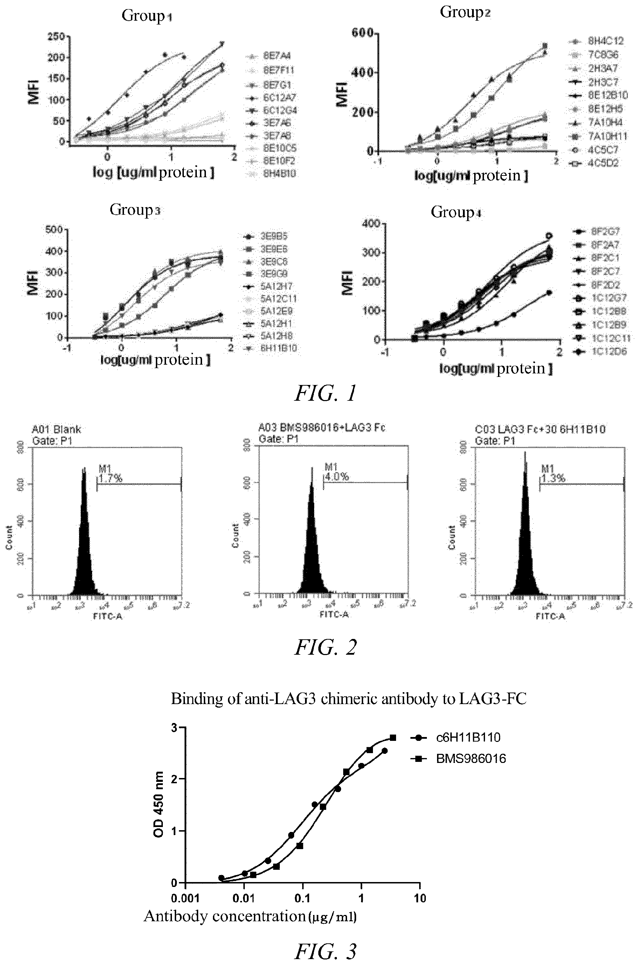

The positive hybridoma clones expressing antibodies screened by ELISA are further determined by a flow cytometry method. First, the CHO-S cells expressing the full-length human LAG3 gene with normal growth are washed twice with PBS (2% FBS), and the cell concentration is adjusted to 5×10⁵/100 µl. A hybridoma supernatant is added and incubated at 4°C for 30 min. The cells are centrifuged at 500 x g for 5 min. No antibody is added to the negative control group. The cells are washed twice with PBS (2% FBS). 1 : 100 diluted goat anti-mouse IgG-FITC (CoWin Biosciences) is added, and incubated at 4°C for 30 min. The cells are washed twice with PBS (2% FBS). The cells are suspended in 200 µl PBS and analyzed by flow cytometry. The results are shown in Figure 1. Most of the positive clones screened by ELISA can bind to LAG3 on the cell surface.

The ability of the antibodies expressed by the hybridoma positive clones screened by ELISA to block the binding of MHC II to LAG3-Fc (Acro) on the cell surface is tested by a flow cytometry method. Daudi cells are washed twice with PBS (2% FBS), and the cell concentration is adjusted to 3 × 10⁵/100 µl. The hybridoma supernatant is mixed with LAG3-Fc at a concentration of 2 µg/ml, then mixed with Daudi cells, and incubated at 4°C for 30 min. The cells are centrifuged at 500 x g for 5 min. Meanwhile, no antibody control is used as a negative control, and the anti-LAG3 antibody BMS986016 is used as a positive control. The cells are washed twice with PBS (2% FBS). 1 : 300 diluted goat anti-human IgG-Fc-FITC (Thermo) is added, and incubated at 4°C for 30 min. The cells are washed twice with PBS (2% FBS). The cells are suspended in 200 µl PBS and analyzed by flow cytometry. The results are shown in Figure 2. Finally, a plurality of monoclonal hybridoma cell lines with the ability to block the binding of MHC II to LAG3-Fc on the cell surface are obtained by screening, and the best monoclonal hybridoma cell line is 6H11B10. 6H11B10 comprises a heavy chain variable region having an amino acid sequence as shown in SEQ ID No: 2 and a nucleotide sequence as shown in SEQ ID No: 3; and a light chain variable region having an amino acid sequence as shown in SEQ ID No: 4 and a nucleotide sequence as shown in SEQ ID No: 5.

### Example 2 Determination of affinity of the anti-LAG3 chimeric antibody

Amplification of mouse light and heavy chain variable regions is performed as described by Anke Krebber et al. (Journal of Immunological Methods 201.1997.35-55). The general method comprises preparing total RNA from the monoclonal hybridoma cell line 6H11B10, and preparing single-stranded cDNA using a reverse transcription kit (TaKaRa). The genes of the heavy chain variable region (SEQ ID No: 3) and the light chain variable region (SEQ ID No: 5) of the antibody are amplified by a PCR reaction using primers that can be complementary to and paired with the mouse light and heavy chain variable regions according to Anke Krebber et al. Then, the genes of the light and heavy chain variable regions of 6H11B10 are fused with those of the heavy chain constant region (SEQ ID No: 15) and the light chain constant region (SEQ ID No: 17) of IgG4, respectively, thereby obtaining the chimeric antibody heavy and light chain genes of 6H11B10.

The chimeric antibody genes of 6H11B10 are respectively transfected into CHO-S cells and expressed to obtain the chimeric antibody protein c6H11B10. The affinity of the chimeric antibody for LAG3 is detected by an ELISA technique. An ELISA plate is coated with an LAG3-Fc recombinant protein at 2 µg/ml (a working volume of 30 µl), and allowed to stand overnight at 4°C. The plate is washed 3 times with PBS containing 0.05% Tween 20 (PBST). The plate is blocked with PBS containing 5% skimmed milk for 1 hour at room temperature. The plate is washed 3 times with PBST, followed by the addition of the gradient-diluted c6H11B10, and allowed to stand at room temperature for 1 hour. Meanwhile, BMS986016 is used as a positive control. The plate is washed 3 times with PBST, and 30 µl of 1 : 4000 diluted horseradish peroxidase-labeled goat anti-human IgG kappa light chain secondary antibody (Millipore) is added thereto, and the plate is allowed to stand at room temperature for 1 hour. The plate is washed 6 times with PBST, and TMB is added thereto for color development. The reaction is stopped with 2M H₂SO₄, and the plate is read at 450 nm with a microplate reader. The results are shown in Figure 3. The anti-LAG3 chimeric antibody c6H11B10 has a strong affinity for LAG3, and at low concentrations, the affinity of c6H11B10 is higher than that of the control antibody BMS986016.

### Example 3 Determination of affinity of the anti-LAG3 chimeric antibody

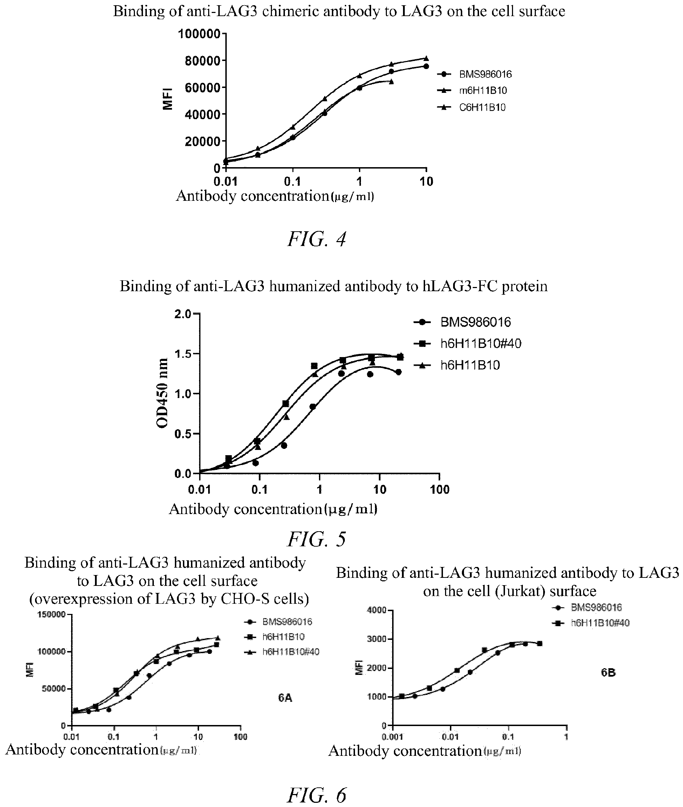

The affinity of the chimeric antibody protein c6H11B10 for LAG3 on the cell surface is determined by a flow cytometry method. The transfected Jurkat cells expressing the full-length human LAG3 gene are taken and washed twice with PBS (2% FBS), and the cell concentration is adjusted to 2×10⁵/100 µl. The gradient-diluted chimeric antibody c6H11B10 and the murine antibody m6H11B10 are added, and incubated at 4°C for 30 min. The cells are centrifuged at 500 x g for 5 min. Meanwhile, BMS986016 is used as a positive control. The cells are washed twice with PBS (2% FBS). For the chimeric antibody and BMS986016, 1 : 300 diluted goat anti-human IgG Fab-FITC (Thermo Fisher) is added; for the murine antibody, 1 : 100 diluted goat anti-mouse IgG-FITC (CoWin Biosciences) is added; and the mixtures are incubated at 4°C for 30 min. The cells are washed twice with PBS (2% FBS). The cells are suspended in 200 µl PBS and analyzed by flow cytometry. The results are shown in Figure 4. The chimeric antibody c6H11B10 has a strong binding to LAG3 on the cell surface, and the affinity of c6H11B10 is better than that of the control antibody BMS986016.

### Example 4 Humanization of the anti-LAG3 murine antibody 6H11B10

For the humanization of the murine antibody 6H11B10, the framework region in the murine antibody 6H11B10 is replaced with the human antibody light chain variable region germline gene IGKV2-28*01 and heavy chain variable region germline gene IGHV3-11*06, and the original complementarity determining regions (CDRs) are retained, thereby obtaining the anti-6H11B10 humanized antibody h6H11B10. h6H11B10 comprises a heavy chain variable region having an amino acid sequence as shown in SEQ ID No: 6, and a light chain variable region having an amino acid sequence as shown in SEQ ID No: 8. h6H11B10 comprises a heavy chain variable region having a gene sequence as shown in SEQ ID No: 7, and a light chain variable region having a gene sequence as shown in SEQ ID No: 9, which are respectively fused with the gene of the heavy chain constant region (SEQ ID No: 15) and the gene of the light chain constant region (SEQ ID No: 17) of IgG4, thereby obtaining the heavy and light chain genes of the anti-LAG3 humanized antibody h6H11B10. The three CDRs of the heavy chain of h6H11B10 are: HCDR1 (SEQ ID No: 18), HCDR2 (SEQ ID No: 19) and HCDR3 (SEQ ID No: 20), respectively; and the three CDRs of the light chain thereof are: LCDR1 (SEQ ID No:22), LCDR2 (SEQ ID No: 24) and LCDR3 (SEQ ID No: 25), respectively.

The anti-LAG3 humanized antibody h6H11B10 is subjected to affinity maturation by a phage display technique, primers with point mutations are designed, and the light chain CDRs and heavy chain CDRs of h6H11B10 are respectively mutated by PCR to obtain a mutation library. The phage display vector is transformed into E. coli TG1 or SS320 cells to generate a phage library. The phage library is screened in two rounds using a streptavidin-coupled magnetic bead M-280 (Thermo Fisher) and a biotin-labeled LAG3 protein. Then, a clone with the highest affinity (h6H11B10#40) is screened out by ELISA. h6H11B10#40 comprises a heavy chain variable region having an amino acid sequence as shown in SEQ ID No: 10, and a light chain variable region having an amino acid sequence as shown in SEQ ID No: 12. h6H11B10#40 comprises a heavy chain variable region having a gene as shown in SEQ ID No: 11, and a light chain variable region having a gene as shown in SEQ ID No: 13, which are respectively fused with the gene of the heavy chain constant region (SEQ ID No: 15) and the gene of the light chain constant region (SEQ ID No: 17) of IgG4, thereby obtaining the heavy and light chain genes of the anti-LAG3 humanized antibody h6H11B10#40. The three CDRs of the heavy chain of h6H11B10#40 are: HCDR1 (SEQ ID No: 18), HCDR2 (SEQ ID No: 19) and HCDR3 (SEQ ID No: 21), respectively; and the three CDRs of the light chain thereof are: LCDR1 (SEQ ID No: 23), LCDR2 (SEQ ID No: 24) and LCDR3 (SEQ ID No: 25), respectively.

### Example 5 Determination of affinity of the anti-LAG3 humanized antibody for LAG3

The genes of the anti-LAG3 humanized antibodies h6H11B10 and h6H11B10#40 are respectively transfected into CHO-S cells and expressed to obtain the antibody proteins h6H11B10 and h6H11B10#40. The affinity of the humanized antibody for LAG3 is detected by an ELISA technique. An ELISA plate is coated with an LAG3-Fc recombinant protein at 2 µg/ml (a working volume of 30 µl), and allowed to stand overnight at 4°C. The plate is washed 3 times with PBS containing 0.05% Tween 20 (PBST). The plate is blocked with PBS containing 5% skimmed milk for 1 hour at room temperature. The plate is washed 3 times with PBST, followed by the addition of the gradient-diluted humanized antibody proteins h6H11B10 and h6H11B10#40, and allowed to stand at room temperature for 1 hour. Meanwhile, BMS986016 is used as a positive control. The plate is washed 3 times with PBST, and 30 µl of 1 : 4000 diluted horseradish peroxidase-labeled goat anti-human IgG kappa light chain secondary antibody (Millipore) is added thereto, and the plate is allowed to stand at room temperature for 1 hour. The plate is washed 6 times with PBST, and TMB is added thereto for color development. The reaction is stopped with 2M H₂SO₄. The plate is read at 450 nm with a microplate reader. The results are shown in Figure 5. The anti-LAG3 humanized antibodies have strong affinity for LAG3, among which, h6H11B10#40 is better than h6H11B10, and both are better than the positive control BMS986016.

The binding of the humanized antibodies to LAG3 on the cell surface is determined by a flow cytometry method. The transfected CHO-S or Jurkat cells expressing the full-length human LAG3 gene are taken and washed twice with PBS (2% FBS), and the cell concentration is adjusted to 5×10⁵/100 µl. 1 : 3 gradient-diluted anti-LAG3 humanized antibody is added, and incubated at 4°C for 30 min. Meanwhile, BMS986016 is used as a positive control. The cells are centrifuged at 500 x g for 5 min. The cells are washed twice with PBS (2% FBS). 1 : 300 diluted goat anti-human IgG Fab-FITC (Thermo Fisher) is added, and incubated at 4°C for 30 min. The cells are washed twice with PBS (2% FBS). The cells are suspended in 200 µl PBS and analyzed by flow cytometry. The results are shown in Figure 6. The anti-LAG3 humanized antibodies have strong affinity for LAG3 on the cell membrane, and both h6H11B10#40 and h6H11B10 are better than the positive control BMS986016.

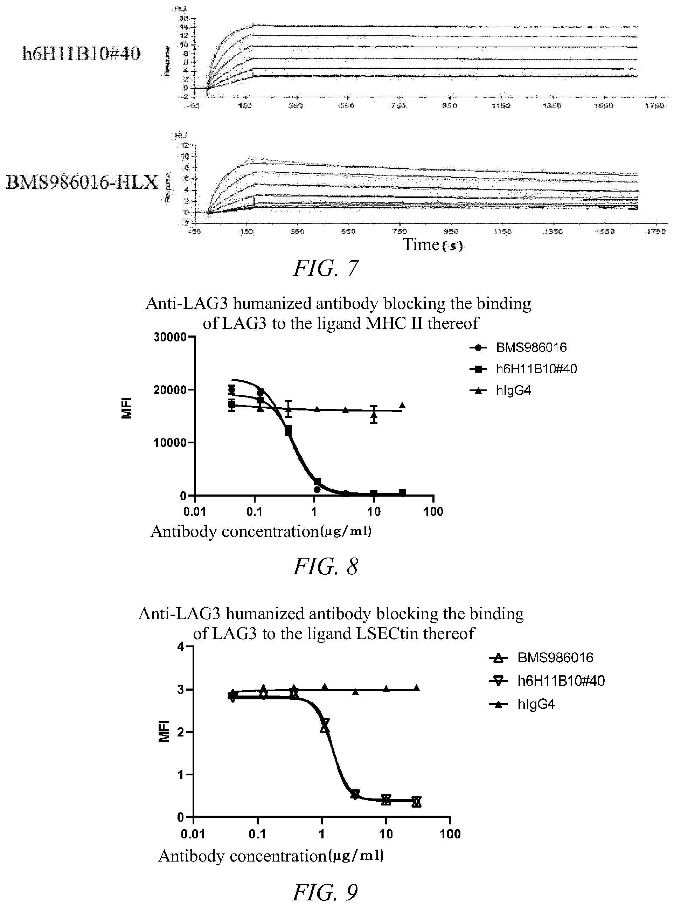

Surface plasmon resonance (SPR) is used to measure the binding affinity and kinetic constants of the anti-LAG3 humanized antibody for the LAG3 protein. The LAG3-Fc protein is first immobilized on a chip, and then the anti-LAG3 humanized antibody h6H11B10#40 and BMS986016 are subjected to gradient dilution from 80 µg/ml to 2.5 µg/ml with an HBSPE buffer, and allowed to flow through the chip respectively. The data obtained from the experiments are analyzed using an evaluation software, and the curve is fitted with a 1 : 1 Langmuir binding model. The kinetic association and dissociation constants and calculated affinity constants (KDs) are shown in Table 1. The results are shown in Figure 7. After affinity maturation, the affinity of the anti-LAG3 humanized antibody h6H11B10#40 for LAG3 is greatly improved, and the association and dissociation constants thereof are better than those of the positive control antibody BMS986016.

**Table 1**

| Samples | ka (1/Ms) | kd (1/s) | KD (M) |
|---|---|---|---|
| BMS986016 | 3.22E+04 | 2.04E-04 | 6.35E-09 |
| h6H11B10#40 | 4.35E+04 | 1.37E-05 | 3.13E-10 |

### Example 6 Ability of the anti-LAG3 humanized antibody to block the binding of LAG3 to the ligand thereof

The ability of the anti-LAG3 humanized antibody to block the binding of MHC II to LAG3-Fc on the Raji cell surface is determined by a flow cytometry method. Raji cells are washed twice with PBS (2% FBS), and the cell concentration is adjusted to 2×10⁵/100 µl. The anti-LAG3 humanized antibody h6H11B10#40 is diluted according to a gradient of 1 : 3, then mixed with LAG3-Fc at a concentration of 2 µg/ml, and then the mixture is placed at room temperature for 30 min. Meanwhile, the hIgG4 protein is used as a negative control, and the anti-LAG3 antibody BMS986016 is used as a positive control. The mixture is then mixed with the Raji cells and incubated at 4°C for 60 min. The cells are centrifuged at 500 x g for 5 min. The cells are washed twice with PBS (2% FBS). 1 : 500 diluted goat anti-human IgG-Fc-Alexa Fluor 488 (Jackson) is added, and incubated at 4°C for 60 min. The cells are washed twice with PBS (2% FBS). The cells are suspended in 200 µl PBS and analyzed by flow cytometry. The results are shown in Figure 8. The anti-LAG3 humanized antibody h6H11B10#40 has the ability to block the binding of MHC II to LAG3-Fc on the cell surface, and has the ability similar to that of BMS986016.

The ability of the humanized antibody to block the binding of LAG3-Fc to the ligand LSECtin thereof is detected by an ELISA technique. An ELISA plate is coated with an LAG3-Fc recombinant protein at 1 µg/ml (a working volume of 100 µl), and allowed to stand overnight at 4°C. The plate is washed 3 times with PBS containing 0.05% Tween 20 (PBST). The plate is blocked with PBS containing 5% skimmed milk for 1 hour at room temperature, and washed 3 times with PBST. The humanized antibody protein h6H11B10#40 is diluted according to a gradient of 1 : 5. Meanwhile, the hIgG4 protein is used as a negative control, and the anti-LAG3 antibody BMS986016 is used as a positive control. In addition, the LSECtin (R&D) is formulated into 1 µg/ml, mixed with the antibody at 1 : 1, and allowed to stand at room temperature for 30 min. Then the mixture is added to an ELISA plate and allowed to stand at room temperature for 1 hour. The plate is washed 3 times with PBST, and 100 µl of 1 : 5000 diluted horseradish peroxidase-labeled mouse anti-His-tag secondary antibody (GenScript) is added thereto, and the plate is allowed to stand at room temperature for 1 hour. The plate is washed 6 times with PBST, and TMB is added thereto for color development. The reaction is stopped with 2M H₂SO₄. The plate is read at 450 nm with a microplate reader. The results are shown in Figure 9. The anti-LAG3 humanized antibody h6H11B10#40 has the ability to block the binding of LAG3-Fc to the ligand LSECtin thereof.

### Example 7 Ability of the anti-LAG3 humanized antibody to block the binding of LAG3 to the ligand thereof

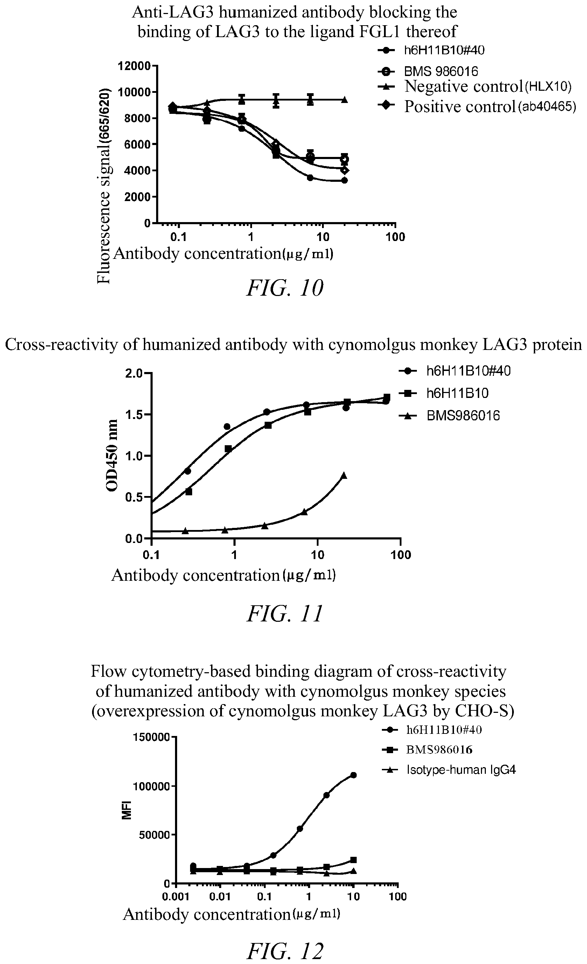

The ability of the anti-LAG3 humanized antibody to block the binding of LAG3 to FGL1 is tested using a commercial kit for determination of the ability of the anti-LAG3 antibody to block the binding of LAG3 to FGL1 (purchased from Cisbio, Cat. No. 63ADK000CB10PEG). In this experiment, 4 µL of a Tag1-LAG3 protein and 4 µL of a Tag2-FGL1 protein are mixed with the test samples diluted at different concentrations. The mixture is reacted at room temperature for 15 minutes, and then 10 µL of pre-mixed anti-Tag1-Tb3+ and anti-Tag2-XL665 are added thereto. The test plate is sealed and reacted at room temperature overnight, and then the fluorescence absorption value at 665/620 nm is read with a microplate reader. The results are shown in Figure 10. The anti-LAG3 humanized antibody h6H11B10#40 has the ability to block the binding of LAG3 to the ligand FGL1 thereof.

### Example 8 Species cross-reactivity of the anti-LAG3 humanized antibody

Recombinant mouse and cynomolgus monkey LAG3-Fc fusion proteins are purchased from Sino Biological. An ELISA plate is coated with an LAG3-Fc recombinant protein at 1 µg/ml (a working volume of 30 µl), and allowed to stand overnight at 4°C. The plate is washed 3 times with PBS containing 0.05% Tween 20 (PBST). The plate is blocked with PBS containing 5% skimmed milk for 1 hour at room temperature. The plate is washed 3 times with PBST, followed by the addition of the gradient-diluted anti-LAG3 humanized antibodies h6H11B10 and h6H11B10#40, and allowed to stand at room temperature for 1 hour. Meanwhile, BMS986016 is used as a positive control. The plate is washed 3 times with PBST, and 30 µl of 1 : 2000 diluted horseradish peroxidase-labeled goat anti-human IgG kappa light chain secondary antibody (millipore) is added thereto, and the plate is allowed to stand at room temperature for 1 hour. The plate is washed 6 times with PBST, and TMB is added thereto for color development. The reaction is stopped with 2M H₂SO₄. The plate is read at 450 nm with a microplate reader. As shown in Figure 11, compared with BMS986016, the anti-LAG3 humanized antibodies h6H11B10 and h6H11B10#40 have higher affinity for cynomolgus monkey LAG3, while BMS986016 has lower affinity for cynomolgus monkey LAG3. Both BMS986016 and h6H11B10 have weak affinity for mouse LAG3 (not shown in the figure).

The binding of the humanized antibodies to cynomolgus monkey LAG3 on the cell surface is determined by a flow cytometry method. The transfected CHO-S cells expressing the full-length cynomolgus monkey LAG3 gene are taken and washed twice with PBS (2% FBS), and the cell concentration is adjusted to 5×10⁵/100 µl. 1 : 3 gradient-diluted anti-LAG3 humanized antibody is added, and incubated at 4°C for 30 min. Meanwhile, BMS986016 is used as a positive control. The cells are centrifuged at 500 x g for 5 min. The cells are washed twice with PBS (2% FBS). 1 : 300 diluted goat anti-human IgG Fab-FITC (Thermo Fisher) is added, and incubated at 4°C for 30 min. The cells are washed twice with PBS (2% FBS). The cells are suspended in 200 µl PBS and analyzed by flow cytometry. The results are shown in Figure 12. The anti-LAG3 humanized antibodies h6H11B10#40 and h6H11B10 have strong affinity for cynomolgus monkey LAG3 on the cell membrane, and both h6H11B10#40 and h6H11B10 are better than the positive control BMS986016.

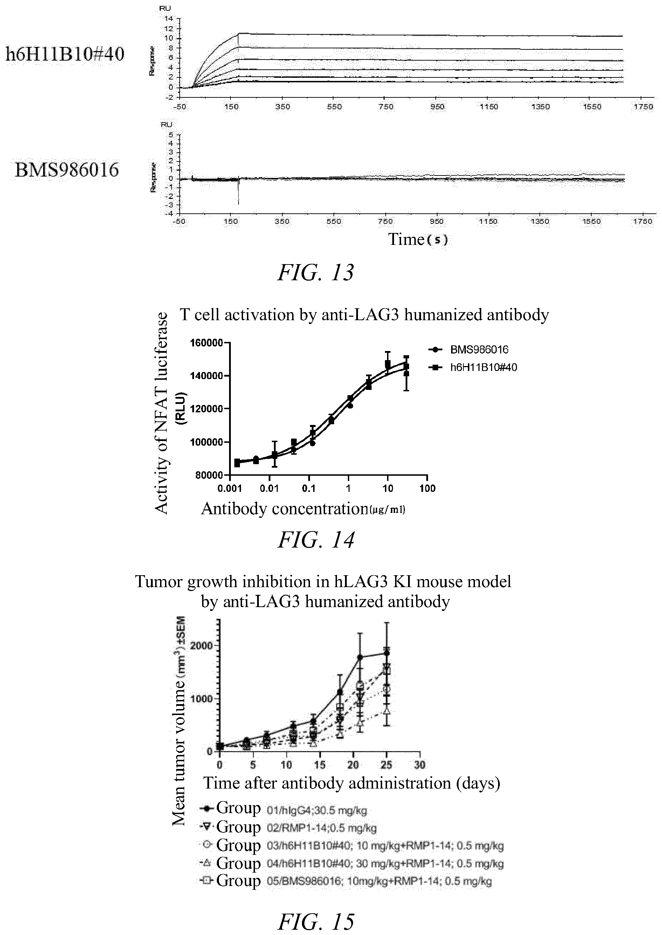

Surface plasmon resonance (SPR) is used to measure the binding affinity and kinetic constants of the anti-LAG3 humanized antibody for the cynomolgus monkey LAG3 protein. The cynomolgus monkey LAG3-Fc protein is first immobilized on a chip, and then the anti-LAG3 humanized antibody h6H11B10#40 and BMS986016 are subjected to gradient dilution from 80 µg/ml to 2.5 µg/ml with an HBSPE buffer, and allowed to flow through the chip respectively. The data obtained from the experiments are analyzed using an evaluation software, and the curve is fitted with a 1 : 1 Langmuir binding model. The kinetic association and dissociation constants and calculated affinity constants (KDs) are shown in Table 2. The results are shown in Figure 13. The anti-LAG3 humanized antibody h6H1 1B10#40 has high affinity for cynomolgus monkey LAG3.

**Table 2**

| Samples | ka (1/Ms) | kd (1/s) | KD (M) |
|---|---|---|---|
| h6H11B10#40 | 2.17E+04 | 3.09E-05 | 1.422E-09 |

### Example 9 Ability of T cell activation by the anti-LAG3 humanized antibody

Raji cells are taken and centrifuged at 500 × g for 5 min, and the cell concentration is adjusted to 1.2×10⁶/ml. SEE (Toxin Technology) is added to a final concentration of 0.024 ng/ml and incubated at 37°C for 30 min. Jurkat cells (Promega) transfected with LAG3 genes and NFAT-Luc reporter genes are taken and centrifuged at 500×g for 5 min, and the cell concentration is adjusted to 1.6×10⁶/ml. 25 µl of the anti-LAG3 humanized antibody diluted according to a gradient of 1 : 3 and 25 µl of BMS986016 (used as a positive control) are respectively taken and mixed with an equal volume of Jurkat cells, and then 25 µl of SEE-treated Raji cells are added thereto. The mixture is incubated at 37°C for 6 hours. Bio-Glo (Promega) is added, and a multi-mode microplate reader is used for reading. The results are shown in Figure 14. The anti-LAG3 humanized antibody activates Jurkat cells to express a luciferase, and the effect thereof is similar to that of BMS986016.

### Example 10 Inhibitory activity of the anti-LAG3 humanized antibody on tumor growth in the hLAG3 KI mouse model

hLAG3 KI mice are used to detect the inhibitory ability of the anti-LAG3 humanized antibody on tumor cell growth *in vivo.* Mouse colon cancer cells M38 are inoculated subcutaneously on the back of the hLAG3 KI mice at 1×10⁶ cells per mouse. When the tumor volume reaches about 80-120 mm³, the intraperitoneal injection of the anti-mouse PD-1 antibody RMP1-14 (CrownBio) (at a dose of 0.5 mg/kg) and the anti-LAG3 humanized antibody h6H11B10#40 (available in two doses, 30 mg/kg and 10 mg/kg, respectively) is started, and is performed twice a week for approximately 2 to 3 weeks. Meanwhile, PBS is injected as a negative control, and the anti-mouse PD-1 antibody RMP1-14 and the anti-LAG3 antibody BMS986016 are injected as positive controls. The size of tumors formed in each mouse is observed twice a week. The tumor volume is calculated as: V (mm³)=0.5×(length (mm)×width (mm)×width (mm)). The results are shown in Figure 15. Compared with the control group injected with PBS, injection of the anti-LAG3 antibody and the anti-mouse PD-1 antibody can significantly inhibit the tumor growth. In the high-dose group, h6H11B10#40 is used in combination with the anti-mouse PD-1 antibody, which has a better efficacy than that of an antibody alone.

### Example 11 Anti-LAG3 antibody-mediated reactivation of exhausted T cells

Human PBMC cells are centrifuged at 500 g for 10 min to remove the supernatant, washed twice with a culture buffer (RPMI-1640 medium+2% FBS), counted, resuspended with a stimulation buffer (RPMI-1640 culture medium+2% FBS+20 ng/mL SEB), and then inoculated into a 96-well cell culture plate (5×10⁵ cells/well). The plate is placed in a 37°C, 7% CO₂ incubator for overnight culture and stimulation. The next day, the desired test samples are subjected to gradient dilution with a culture buffer. In IL-2 and IFN-γ test experiments, the test groups include: the constant-gradient-diluted h6H11B10#40 sample group (the highest test concentration being 10 µg/mL), and the constant-gradient-diluted HLX10 group (the highest test concentration being 10 µg/mL). In a combined administration group, h6H11B10#40 is diluted with an HLX10 solution at different concentrations (1 µg/mL, 0.1 µg/mL, 0.01 µg/mL and 0.001 µg/mL). The diluted test samples are successively added to the PBMC (which has been stimulated overnight), placed in a 37°C, 7% CO₂ incubator, and incubated for another 2-3 days. The supernatant is taken, and the IL-2 and IFN-γ kits are used to determine the amount of IL-2 and IFN-γ released in each test group.

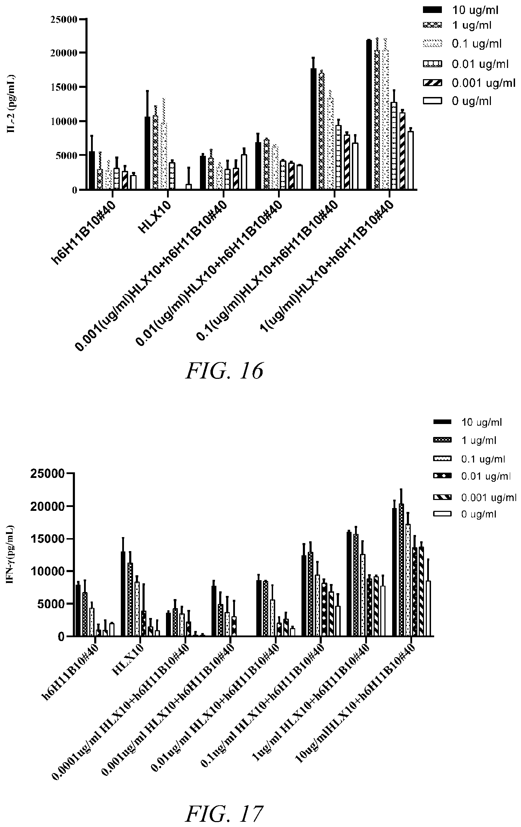

As shown by data in Figures 16 and 17, the amount of IL-2 and IFN-γ in the group treated with h6H11B10#40 or HLX10 alone is lower. When the HLX10 reaches a concentration of about 1 µg/mL and is administered in combination with h6H11B10#40, the release amount of IL-2 and IFN-γ is higher than that of the h6H11B10#40 single agent group and the HLX10 single agent group. In a combined administration group (the HLX10 concentration being 1 µg/mL or 0.1 µg/mL), h6H11B10#40 shows a concentrationdependent effect. This *in vitro* experiment demonstrates that the combined administration of h6H11B10#40 and HLX10 can reactivate exhausted T cells and increase the release of cytokines.

### Example 12 Efficacy evaluation in the hLAG-3/hPD-1 transgenic mice MC38 or A20 subcutaneously transplanted tumor model

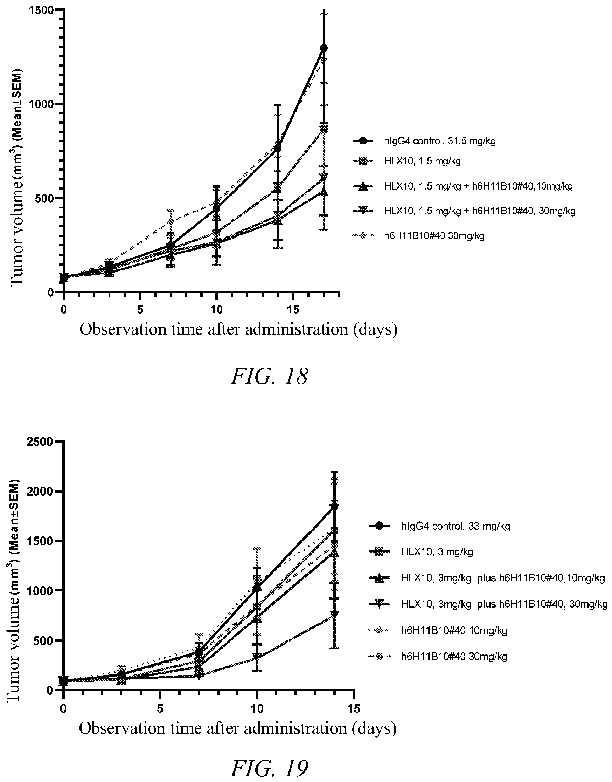

MC38 cells are cultured in a growth medium (RPMI-1640+10% FBS). Cells in logarithmic growth phase are collected before inoculation, and resuspended in PBS and Matrigel (0.1 mL/mouse) for subcutaneous inoculation of mice. The experimental mice to be inoculated are 6-8 weeks old, and MC38 cells (1×10⁶ cells/mouse) are subcutaneously inoculated on the posterior-lower part of the right back. When the average tumor volume is 78.03 mm³, the mice are randomly divided into groups according to tumor size. The coefficient of variation (CV) of tumor volume among each group is calculated with the formula CV = SD/MTV × 100%, which should be less than 40%. The day of grouping is defined as day 0, and administration begins on day 0. The clinical symptoms observed during the experiment are all recorded in the raw data. The calculation formula of tumor volume is as follows: tumor volume (mm³) = 1/2 × (a × b²) (wherein, a represents the long diameter, and b represents the short diameter). The results are shown in Figure 18. In the MC38 model, the mice are euthanized as the tumor volume in the control mouse group reaches 2500 mm³ on day 17 of administration during the experiment. According to the data analysis of D17, for the single agent groups HLX10 and h6H11B10#40, TGI is 35.22% and 4.88%, respectively. In a combined administration group, when the dose of HLX10 is 1.5 mg/kg and the dose of h6H11B10#40 is set to 10 mg/kg and 30 mg/kg, respectively, TGI% is 62.2% and 56.7%, respectively, which are higher than those of the single agent groups. The data shows that the combined administration of h6H11B10#40 and HLX10 has better tumor-inhibiting effects than a single agent.

A20 cells are cultured in the culture medium RPMI-1640+10% FBS. The A20 cells in exponential growth phase are collected, and then resuspended in PBS to an appropriate concentration (0.1 mL/mouse) for subcutaneous inoculation of mice. Experimental mice are subcutaneously inoculated with the A20 cells (5×10⁵ cells/mouse) on the right anterior scapula. When the average tumor volume reaches 90.44 mm³, the mice are randomly divided into groups according to tumor size. The coefficient of variation (CV) of tumor volume among each group is calculated with the formula CV = SD/MTV × 100%, which should be less than 40%. The day of grouping is defined as day 0, and administration begins on day 0. In the A20 model, the mice are euthanized as the tumor volume is greater than 2500 mm³ on day 14 of administration, which causes reduced number of mice. TGI analysis is performed on day 14, and as shown in Figure 19, for single agent groups HLX10 (3 mg/kg), h6H11B10#40 (10 mg/kg) and h6H11B10#40 (30 mg/kg), TGI is 13.36%, 12.83% and 22.20%, respectively. In a combined administration group, when the dose of HLX10 is 3 mg/kg and the doses of h6H11B10#40 are 10 mg/kg and 30 mg/kg, respectively, TGI% is 25.9% and 62.4%, respectively, which are better than those of the single agent groups, indicating that the combined administration group has tumor-inhibiting effects.

It should be understood that after reading the above content of the present invention, those skilled in the art can make various changes or modifications to the present invention, and these equivalent forms also fall within the scope defined by the appended claims of this application.

### Sequence Listing

SEQ ID NO: 1 (an amino acid sequence of LAG3-His) 
LQPGAEVPVVWAQEGAPAQLPCSPTIPLQDLSLLRRAGVTWQHQPDSGPPAAAPGHPLAPGPHPAAPSSW
GPRPRRYTVLSVGPGGLRSGRLPLQPRVQLDERGRQRGDFSLWLRPARRADAGEYRAAVHLRDRALSCR
LRLRLGQASMTASPPGSLRASDWVILNCSFSRPDRPASVHWFRNRGQGRVPVRESPHHHLAESFLFLPQV
SPMDSGPWGCILTYRDGFNVSIMYNLTVLGLEPPTPLTVYAGAGSRVGLPCRLPAGVGTRSFLTAKWTPPG
GGPDLLVTGDNGDFTLRLEDVSQAQAGTYTCHIHLQEQQLNATVTLAIITVTPKSFGSPGSLGKLLCEVTP
VSGQERFVWSSLDTPSQRSFSGPWLEAQEAQLLSQPWQCQLYQGERLLGAAVYFTELSSPGAQRSGRAP
GALPAGHLGSGHHHHHH
SEQ ID NO: 2 (a heavy chain variable region amino acid sequence of 6H11B10) 
EVQVVESGGGLVKPGGSLKLSCAASGFTFSDYYMFWVRQTPEERLEWVATINDGGDYTNYPDSVKGRFT
ISRDNDKNKLYLQMNSLKSEDTAMYYCARESGIYYDYASFGYWGQGTLVTVSS
SEQ ID NO: 3 (a heavy chain variable region nucleotide sequence of 6H11B10) 
gaggtgcaggtggtggagtctgggggaggtttagtgaagcctggagggtccctgaaactctcctgtgcagcgtctggattcactttcagtgactattacatgttttgggtt
cgccagactccggaagagaggctggagtgggtcgcaaccattaatgatggtggtgattacaccaactatccagacagtgtgaaggggcgattcaccatctccagag
acaatgacaagaacaaactgtacctgcaaatgaacagtctgaagtctgaggacacagccatgtattactgtgcaagagagagtgggatctactatgattacgcttcgttt
ggttactggggccaggggactctggtcactgtctct
SEQ ID NO: 4 (a light chain variable region amino acid sequence of 6H11B10) 
DIVMTQAAFSNPVTLGTSASISCRSSKSLLYSNGITYLYWYLQKPGQSPQLLIYQMSNLASGVPDRFSSSGS
GTDFTLRISRVEAEDVGVYYCAQNLELPWTFGGGTKLEIKRNL
SEQ ID NO: 5 (a light chain variable region nucleotide sequence of 6H11B10) 
gatattgtgatgacccaggctgcattctccaatccagtcactcttggaacatcagcttccatctcctgcaggtctagtaagagtctcttatatagtaatggcatcacttatttg
tattggtatctgcagaagccaggccagtctcctcagctcctgatttatcagatgtccaaccttgcctcaggagtcccagacaggttcagtagcagtgggtcaggaactg
atttcacactgagaatcagcagagtggaggctgaggatgtgggtgtttattactgtgctcaaaatctagaacttccgtggacgttcggtggaggcaccaagctggaaat
caaacgtaatctg
SEQ ID NO: 6 (a heavy chain variable region amino acid sequence of h6H11B10) 
QVQLVESGGGLVKPGGSLRLSCAASGFTFSDYYMFWIRQAPGKGLEWVATINDGGDYTNYPDSVKGRFT
ISRDNAKNSLYLQMNSLRAEDTAVYYCARESGIYYDYASFGYWGQGTLVTVSS
SEQ ID NO: 7 (a heavy chain variable region nucleotide sequence of h6H11B10) 
caggtgcagctggtggagagcggaggaggactggtgaagccaggaggatctctgaggctgtcctgcgccgctagcggcttcaccttttctgactactatatgttctgg

atcagacaggctccaggcaagggactggagtgggtggctaccatcaacgacggcggcgattacacaaattatcctgactccgtgaagggcaggtttaccatctctcg

ggataacgccaagaattccctgtacctgcagatgaacagcctgagagccgaggacacagccgtgtactattgtgcccgcgagtctggcatctactatgattacgcttc

cttcggctattggggccagggcaccctggtgacagtgtcctcc
SEQ ID NO: 8 (a light chain variable region amino acid sequence of h6H11B10) 
DIVMTQSPLSLSVTPGEPASISCRSSKSLLYSNGITYLYWYLQKPGQSPQLLIYQMSNLASGVPDRFSGSGS
GTDFTLKISRVEAEDVGVYYCAQNLELPWTFGGGTKLEIKRNL
SEQ ID NO: 9 (a light chain variable region nucleotide sequence of h6H11B10) 
gacatcgtgatgacccagtccccactgtctctgtccgtgacaccaggagagcctgctagcatctcttgcagatccagcaagtccctgctgtatagcaacggcatcacct
acctgtattggtacctgcagaagccaggccagagcccccagctgctgatctaccagatgtctaatctggcttccggagtgcctgacaggttctccggaagcggatctg
gaaccgacttcaccctgaagatctctcgggtggaggccgaggatgtgggcgtgtactattgtgctcagaacctggagctgccatggaccttcggcggaggaacaaa
gctggagatcaagcgcaatctg
SEQ ID NO: 10 (a heavy chain variable region amino acid sequence of h6H11B10#40) 
QVQLVESGGGLVKPGGSLRLSCAASGFRFSDYYMFWIRQAPGKGLEWVATINDGGDYTNYPDSVKGRFT
ISRDNAKNSLYLQMNSLRAEDTAVYYCARESGIYYDWASFGYWGQGTLVTVSS
SEQ ID NO: 11 (a heavy chain variable region nucleotide sequence of h6H11B10#40) 
caggtgcagctggtggagagcggaggaggactggtgaagccaggaggatctctgaggctgtcctgcgccgctagcggcttccggttttctgactactatatgttctgg

atcagacaggctccaggcaagggactggagtgggtggctaccatcaacgacggcggcgattacacaaattatcctgactccgtgaagggcaggtttaccatctctcg

ggataacgccaagaattccctgtacctgcagatgaacagcctgagagccgaggacacagccgtgtactattgtgcccgcgagtctggcatctactatgattgggcttc

cttcggctattggggccagggcaccctggtgacagtgtcctcc
SEQ ID NO: 12 (a light chain variable region amino acid sequence of h6H11B10#40) 
DIVMTQSPLSLSVTPGEPASISCRSSKSLKYSNGITYLYWYLQKPGQSPQLLIYQMSNLASGVPDRFSGSGS
GTDFTLKISRVEAEDVGVYYCAQNLELPWTFGGGTKLEIKRNL
SEQ ID NO: 13 (a light chain variable region nucleotide sequence of h6H11B10#40) 
gacatcgtgatgacccagtccccactgtctctgtccgtgacaccaggagagcctgctagcatctcttgcagatccagcaagtccctgaagtatagcaacggcatcacc

tacctgtattggtacctgcagaagccaggccagagcccccagctgctgatctaccagatgtctaatctggcttccggagtgcctgacaggttctccggaagcggatct

ggaaccgacttcaccctgaagatctctcgggtggaggccgaggatgtgggcgtgtactattgtgcgcagaacctggagctgccatggaccttcggcggaggaacaa

agctggagatcaagcgcaatctg
SEQ ID NO: 14 (a heavy chain constant region amino acid sequence) 
ASTKGPSVFPLAPCSRSTSESTAALGCLVKDYFPEPVTVSWNSGALTSGVHTFPAVLQSSGLYSLSSVVTVP
SSSLGTKTYTCNVDHKPSNTKVDKRVESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK
SEQ ID NO: 15 (a heavy chain constant region nucleotide sequence) 
gccagcacaaagggcccatcggtcttcccgctagcaccctgctccaggagcacctccgagagcacagccgccctgggctgcctggtcaaggactacttccccgaa

ccggtgacggtgtcgtggaactcaggcgccctgaccagcggcgtgcacaccttcccggctgtcctacagtcctcaggactctactccctcagcagcgtggtgaccgt

gccctccagcagcttgggcacgaagacctacacctgcaacgtagatcacaagcccagcaacaccaaggtggacaagagagttgagtccaaatatggtcccccatg

cccaccatgcccagcacctgagttcctggggggaccatcagtcttcctgttccccccaaaacccaaggacactctcatgatctcccggacccctgaggtcacgtgcgt

ggtggtggacgtgagccaggaagaccccgaggtccagttcaactggtacgtggatggcgtggaggtgcataatgccaagacaaagccgcgggaggagcagttca

acagcacgtaccgtgtggtcagcgtcctcaccgtcctgcaccaggactggctgaacggcaaggagtacaagtgcaaggtctccaacaaaggcctcccgtcctccat

cgagaaaaccatctccaaagccaaagggcagccccgagagccacaggtgtacaccctgcccccatcccaggaggagatgaccaagaaccaggtcagcctgacc

tgcctggtcaaaggcttctaccccagcgacatcgccgtggagtgggagagcaatgggcagccggagaacaactacaagaccacgcctcccgtgctggactccgac

ggctccttcttcctctacagcaggctaaccgtggacaagagcaggtggcaggaggggaatgtcttctcatgctccgtgatgcatgaggctctgcacaaccactacaca

cagaagagcctctccctgtctctgggtaaa
SEQ ID NO: 16 (a light chain constant region amino acid sequence) 
AAPSVFIFPPSDEQLKSGTASVVCLLNNFYPREAKVQWKVDNALQSGNSQESVTEQDSKDSTYSLSSTLTL
SKADYEKHKVYACEVTHQGLSSPVTKSFNRGEC
SEQ ID NO: 17 (a light chain constant region nucleotide sequence) 
gctgcaccatctgtcttcatcttcccgccatctgatgagcagttgaaatctggaactgcctctgttgtgtgcctgctgaataacttctatcccagagaggccaaagtacagt

ggaaggtggataacgccctccaatcgggtaactcccaggagagtgtcacagagcaggacagcaaggacagcacctacagcctcagcagcaccctgacgctgagc

aaagcagactacgagaaacacaaagtctacgcctgcgaagtcacccatcagggcctgagctcgcccgtcacaaagagcttcaacaggggagagtgt
SEQ ID NO: 18 (HCDR1 amino acid sequences of h6H11B10 and h6H1 1B10#40) DYYMF
SEQ ID NO: 19 (HCDR2 amino acid sequences of h6H11B10 and h6H11B10#40) TINDGGDYTNYPDSVKG
SEQ ID NO: 20 (an HCDR3 amino acid sequence of h6H11B10) ESGIYYDYASFGY
SEQ ID NO: 21 (an HCDR3 amino acid sequence of h6H11B10#40) ESGIYYDWASFGY
SEQ ID NO: 22 (an LCDR1 amino acid sequence of h6H11B10) RSSKSLLYSNGITYLY
SEQ ID NO: 23 (an LCDR1 amino acid sequence of h6H11B10#40) RSSKSLKYSNGITYLY
SEQ ID NO: 24 (LCDR2 amino acid sequences of h6H11B10 and h6H1 1B10#40) QMSNLAS
SEQ ID NO: 25 (LCDR3 amino acid sequences of h6H11B10 and h6H1 1B10#40) AQNLELPWT

## Claims

1. An anti-LAG3 monoclonal antibody, wherein the anti-LAG3 monoclonal antibody comprises a heavy chain variable region comprising HCDR1, HCDR2 and HCDR3 identical to CDR sequences of the heavy chain variable region as shown in SEQ ID NO: 2, 6 or 10, and a light chain variable region comprising LCDR1, LCDR2 and LCDR3 identical to CDR sequences of the light chain variable region as shown in SEQ ID NO: 4, 8 or 12.

2. The anti-LAG3 monoclonal antibody of claim 1, wherein the anti-LAG3 monoclonal antibody comprises:
1) heavy chain complementarity determining regions: HCDR1 having an amino acid sequence as shown in SEQ ID No: 18, HCDR2 having an amino acid sequence as shown in SEQ ID No: 19, and HCDR3 having an amino acid sequence as shown in SEQ ID No: 20 or 21; and
2) light chain complementarity determining regions: LCDR1 having an amino acid sequence as shown in SEQ ID No: 22 or 23, LCDR2 having an amino acid sequence as shown in SEQ ID No: 24, and LCDR3 having an amino acid sequence as shown in SEQ ID No: 25.

3. The anti-LAG3 monoclonal antibody of claim 2, wherein the anti-LAG3 monoclonal antibody comprises a heavy chain variable region having an amino acid sequence as shown in SEQ ID NO: 2, 6 or 10, or a sequence with at least 85% homology thereto; and a light chain variable region having an amino acid sequence as shown in SEQ ID NO: 4, 8 or 12, or a sequence with at least 85% homology thereto.

4. The anti-LAG3 monoclonal antibody of claim 2, wherein the anti-LAG3 monoclonal antibody comprises a heavy chain composed of a heavy chain variable region having an amino acid sequence as shown in SEQ ID NO: 2, 6 or 10, or a sequence with at least 85% homology thereto, and a heavy chain constant region as shown in SEQ ID NO: 14; and a light chain composed of a light chain variable region having an amino acid sequence as shown in SEQ ID NO: 4, 8 or 12, or a sequence with at least 85% homology thereto, and a light chain constant region as shown in SEQ ID NO: 16.

5. The anti-LAG3 monoclonal antibody of claim 1, wherein the anti-LAG3 monoclonal antibody is a full-length sequence of the antibody or an antigen-binding fragment comprising the anti-LAG3 antibody, wherein the antigen-binding fragment of the anti-LAG3 antibody is Fab, Fab', F(ab')2, Fv or scFv.

6. A nucleotide molecule, wherein the nucleotide molecule encodes the anti-LAG3 monoclonal antibody of any one of claims 1 to 5.

7. The nucleotide molecule of claim 6, wherein in the nucleotide molecule, the nucleotide sequence encoding the heavy chain variable region of the anti-LAG3 monoclonal antibody is as shown in SEQ ID NO: 3, 7 or 11, and the nucleotide sequence encoding the light chain variable region of the anti-LAG3 monoclonal antibody is as shown in SEQ ID NO: 5, 9 or 13.

8. An expression vector, wherein the expression vector contains the nucleotide molecule of claim 6 or 7.

9. The expression vector of claim 8, wherein the expression vector is selected from one or more of pHLX101, pEE14.4, pCHO 1.0 or pcDNA3.1.

10. A host cell, wherein the host cell contains the expression vector of claim 8 or 9.

11. The host cell of claim 10, wherein the host cell is selected from one or more of COS, CHO, an HeLa cell line, a myeloid cell line such as an SP2/0 cell line, NS0, sf9, sf21, DH5α, BL21(DE3) or E.coli TG1, a YB2/0 cell line and a transformed B cell or a hybridoma cell.

12. A method for preparing the anti-LAG3 monoclonal antibody of any one of claims 1 to 5, comprising the following steps:
a) culturing the host cell of claim 10 or 11 to express the anti-LAG3 monoclonal antibody under an expression condition; and
b) isolating and purifying the anti-LAG3 monoclonal antibody obtained in step a).

13. A composition, wherein the composition comprises the anti-LAG3 monoclonal antibody of any one of claims 1 to 5 and a pharmaceutically acceptable carrier.

14. Use of the anti-LAG3 monoclonal antibody of any one of claims 1 to 5 or the composition of claim 13 in the preparation of an LAG3 molecule blocking drug, especially in the preparation of a drug for resisting tumors, treating autoimmune diseases, and treating infectious diseases and/or resisting transplantation rejection reactions.

15. The use of claim 14, wherein the anti-LAG3 monoclonal antibody is used alone or in combination with other drugs for resisting tumors which are selected from an antibody drug, such as an anti-PD-1 monoclonal antibody, or a small-molecule drug for resisting tumors, such as paclitaxel and 5-fluorouracil.
